# EUROPEAN PATENT APPLICATION

(11) **EP 0 592 813 A1**
(43) Date of publication of application: **20.04.1994**
(21) Application number: 93114481.0
(22) Date of filing: 09.09.1993
(51) Int. Cl.: A61F 2/16

(54) **Intraocular lens**

(30) Priority: 30.09.1992 JP 68248/92 U
(71) Applicant: HOYA CORPORATION, Shinjuku-ku Tokyo (JP)
(72) Inventor: Shimizu, Hiroyuki, Yono-shi, Saitama-ken (JP); Tsurimaki, Yutaka, Oyama-shi, Tochigi-ken (JP); Daicho, Masanori, Kodama-gun, Saiatama-ken (JP)
(74) Representative: Türk, Gille, Hrabal, Leifert

(57) **Abstract**

An intraocular lens (10) is disclosed which has an optic (11), a haptic (12) and at least one connecting section (13), said haptic encircling the periphery of the side rim of the optic in a continuous annular shape of substantially 360⁰C along the side rim of said optic. At least one pair of grips (14a,14b) for gripping said haptic in an inwardly deflecting manner are provided at points on said haptic.

## Description

### BACKGROUND OF THE INVENTION

### Field of the Invention

The present invention relates to an intraocular lens.

### Description of the Prior Art

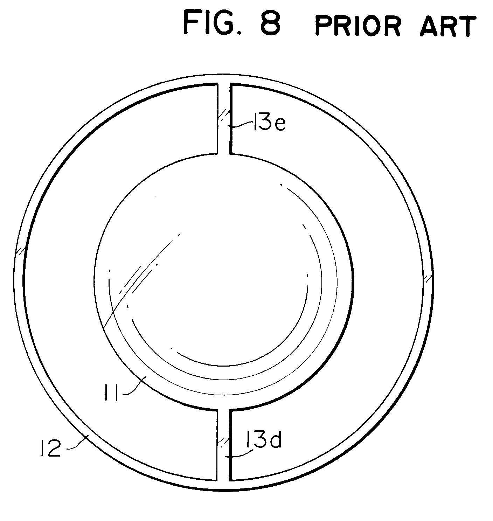

The history of intraocular lenses began in 1949, when Ridley made the first intraocular insertion of a simply formed PMMA lens. For a short time thereafter, the trend was towards intraocular lenses which could be fixed to the iris with a clip after intracapsular extraction which involves removal of a clouded human crystal lens together with the lenticular capsule. Due to the progress of techniques accumulating from a great deal of trial and error, the style of operation soon shifted to a method in which the lenticular capsule was retained and only the contents of the crystal lens were taken, i.e., extracapsular extraction. Along with the evolution of this surgical technique, lens designs also underwent a transformation to simpler and more easily manageable types. In recent years, it has become common to implant open loop-type one-piece lenses which have two(2) J- or C-shaped haptics on the peripheral edge of the optic, into the capsule of the intraocular posterior chamber or ciliary fossa. However, in order to achieve sufficient optical properties, it has always been endeavored to fix the intraocular lenses at the center to prevent deviation or inclining, and to maintain it at a prescribed position with long-term stability. In order to meet these requirements, lenses began to be sought to have a design wherein the haptic is shaped to press uniformly onto the peripheral tissue of the inside of the capsule. As an example of a model which achieves this purpose, a disc lens in which the haptic encircles the periphery of the optic by 360° is disclosed in U.S. Patent Specification No. 4,878,911. A representative example of such a disc lens is shown in Fig. 8. The disc lens shown in Fig. 8 comprises an optic 11, a haptic 12, and connecting sections 13c, 13d connecting the optic 11 and the haptic 12.

Nevertheless, when the above mentioned disc lens in Fig. 8 is intraocularly implanted, if it is attempted to insert the lens through a small incision then the haptic 12 of the lens must be modified to match the measurements of the incision, but since the above mentioned haptic 12 is constructed in a 360° annular shape, it slips easily and it is very difficult to grip the outside surface of the haptic to deflect it towards the middle of the lens. As a result, there has been a considerable amount of physical burden on the surgeon during such operations.

### SUMMARY OF THE INVENTION

The present invention was accomplished in order to overcome the above mentioned disadvantages, and its purpose is to provide an intraocular lens whose advantages include being able to be maintained in a prescribed intraocular position with proper long-term stability, and being easily deformable to measurements adapted to insertion through a small incision, in order to relieve the physical burden on the surgeon.

The present invention was accomplished in order to achieve the above mentioned object, and the present invention is characterized in that the intraocular lens is provided with an optic, a haptic and at least one connecting section connecting the optic and the haptic,
the haptic encircles the periphery of the rim of the optic in a continuous annular shape of substantially 360° along the rim of said optic,
wherein at least one pair of grips for gripping said haptic in an inwardly deflecting manner are provided at points on said haptic symmetrical to the optical axis of said optic or at points on the haptic symmetrical to the optical axis surface of said optic.

### BRIEF EXPLANATION OF DRAWINGS

Fig. 1A is a top view of the first example of an intraocular lens according to the present invention, Fig. 1B is a cross-sectional view along the line I-I of Fig. 1A, Fig. 2 is a rough perspective drawing which shows the intraocular lens in Fig. 1 being gripped with forceps, and Figs. 3A and 3B are rough drawings of the intraocular lens in Fig. 1 being intracapsularly implanted and fixed.

Fig. 4A is a top view of a second example of an intraocular lens according to the present invention, and Fig. 4B is a cross-sectional view along the line II-II of Fig. 4A.

Fig. 5 is a top view showing a third example of an intraocular lens according to the present invention.

Fig. 6 is a top view showing a fourth example thereof.

Fig. 7 is a top view showing a fifth example thereof.

### DETAILED DESCRIPTION OF THE INVENTION

An intraocular lens according to the present invention has its grips constructed on the side surface of the haptic at positions symmetrical to the optical axis or the optical axis surface, and therefore it may be gripped using handling tools such as forceps or the like, enabling easy deflecting of the haptic for intraocular insertion.

Furthermore, since the haptic forms a continuous annular shape of substantially 360°, it is fixed at the center upon intraocular insertion and even if age or shrinkage of the lenticular capsule occurs after the operation, an average force is applied to the haptic, and thus the center does not move. In addition, there is no deformation of the haptic at this time, allowing it to maintain its circular shape and not slide out from the capsule.

An explanation of the present invention will now be provided based on the Examples, with reference to the drawings, but the present invention is not limited to the Examples.

### Example 1

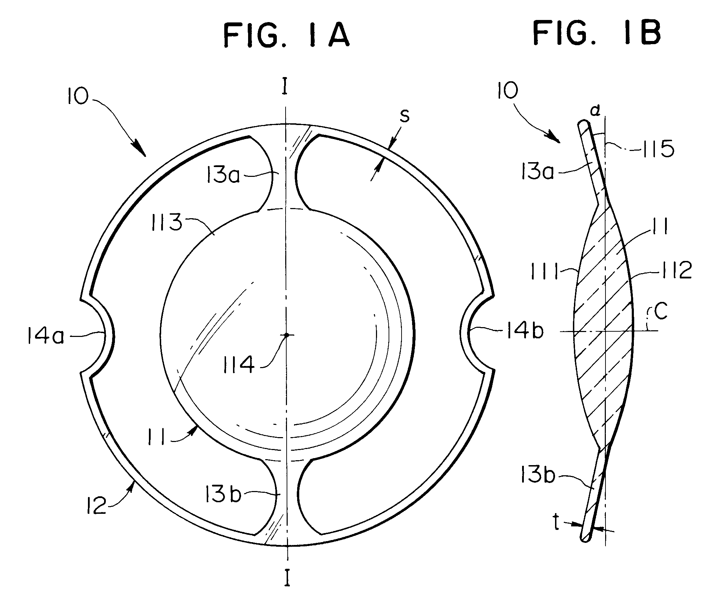

The intraocular lens 10 according to the present embodiment, as shown in Figs. 1A and 1B, is basically constructed with an optic 11, a haptic 12, connecting sections 13 (13a, 13b) and grips 14 (14a, 14b). Also, this intraocular lens 10 is integrally constructed with polymethyl methacrylate (hereunder referred to as PMMA).

The above mentioned optic 11 is a biconvex lens constructed in the form of a circle with a diameter of, for example, about 6.0 mm when considered as a plane, with a cross-sectional shape wherein both optical surfaces 111 (lens front: surface which becomes the cornea side after intraocular implantation) and 112 (lens back: surface which becomes the retina side after intraocular implantation) form convex surfaces. The optic 11 is preferably constructed so as to provide refractivity at the principal point in the range of +4D ∼ +40D in terms of intraocular values.

A pair of connecting sections 13a and 13b, which are symmetrical with respect to the lens center 114, extend out about 2.0 mm from the side rim 113 of the above mentioned optic 11, in a diametral direction from the optic, while the above mentioned connecting sections 13a and 13b, as shown in Fig. 1B, are constructed so as to be inclined at an angle α with respect to the plane 115 which passes through the lens center of the optic 11 and is perpendicular to the optical axis C. This angle α is set to allow the intraocular lens to be implanted and fixed at a prescribed intraocular position, but its values differ from fixation into the lenticular capsule and into the ciliary fossa, and also depending upon the type, curvature, etc. of the optical surface of the lens, but normally in most cases an angle of between 5° and 15° is preferred. For example, the angle α formed by the connecting sections 13a and 13b with the intraocular lens 10 according to the present example is 10°.

Furthermore, since the shape of the plane surface of the above mentioned connecting sections 13a and 13b is, as shown in Fig. 1A, extended in both directions to increase the area (or volume) of the periphery of the points of contact with the optic 11 and the haptic 12, respectively, the connections between the three(3) sections, the optic, the connecting section and the haptic, are stronger and the shape-retention properties of the entire intraocular lens are reinforced.

The haptic 12 is a flexible rod-shaped loop filament having a roughly rectangular cross-sectional shape, and it is connected with the above mentioned optic 11 via the above mentioned connecting sections 13a and 13b. Also, the haptic 12 is constructed concentrically with the optic 11 around the lens center 114 when viewed as a plane, and is constructed on a plane perpendicular to the optical axis. As a result, the haptic 12, which is connected with the optic 11 via the connecting sections 13a and 13b, continuously encircles the periphery of the optic 11 in a substantially 360° arc. Therefore, as described later, the intraocular lens 10 according to the present example is stably maintained when intraocularly implanted.

In addition, regarding the roughly rectangular cross-section of the above mentioned haptic 12, its plane visual diameter (width) and cross-sectional size (thickness) are 0.12 mm and 0.16 mm, respectively, in the present example. Here, "width" refers to the measurements of the haptic shown in the top view of the intraocular lens, for example the measurements indicated by "s" in Fig. 1 (A). Also, "thickness" refers to the measurements of the haptic shown in the cross-sectional or side view of the intraocular lens, for example the measurements indicated by "t" in Fig. 1 (B). The preferred range of the width (s) and the width-to-thickness ratio (s/t) of the haptic 12 according to the present example is a width of 0.05 ∼ 0.5mm and a width-to-thickness ratio of 0.5 ∼ 1.5. If the haptic is in a range which meets the above mentioned conditions, then, as shall be described later, it is possible to achieve sufficient flexibility to easily deflect the haptic 12 by pinching the grips 14a and 14b, and shape-stability after pinching.

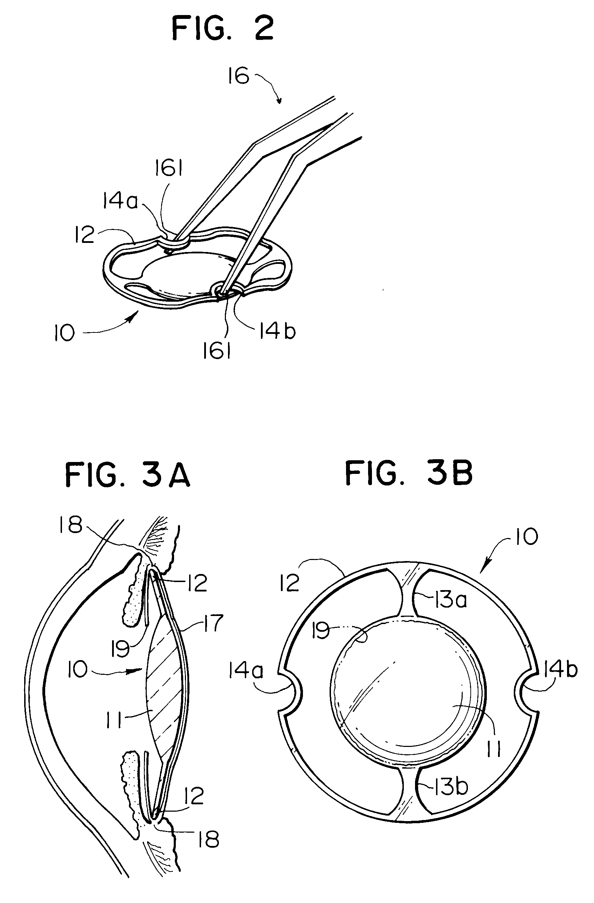

The haptic 12 of the intraocular lens 10 of the present example comprises a pair of semi-circular concave grips 14a and 14b which are constructed so as to form openings facing outward along the diameter perpendicular to the diameter connecting between the two plane visual connecting sections 13a and 13b. When viewed as a plane, as shown in Fig. 2, this pair of grips 14a and 14b provided on the haptic 12 are necessary, for example, to grip the intraocular lens 10 using handling tools such as forceps or the like, and since the haptic 12 deflects when the grips 14a and 14b of the intraocular lens 10 are pinched with the object holding section 161 at the tips of the forceps 16, the shape may be easily varied to facilitate intraocular insertion. As a result, intraocular insertion is possible even through a small incision.

The diameter of the grips 14a and 14b according to the present example is to be appropriately chosen depending upon the type, size, etc. of the handling tool, but it is preferably 0.2 ∼ 3 (mm).

An explanation will now be given regarding the state after implantation of the above mentioned intraocular lens 10 at a prescribed intraocular position. Figs. 3 (A) and (B) show the states of the lens implanted and fixed into the lenticular capsule using the anterior capsular incision method. The haptic 12 of the intraocular lens 10 contacts with the intracapsular tissue 18 of the lenticular capsule 17 at a full circumference of 360°, except for the sections at the grips 14a and 14b. As a result, it may be easily fixed in a proper manner at the center of the intraocular optical axis, and since an average force is applied to the entire body of the haptic 12, no deviation of the optical center occurs even if the lenticular capsule contracts due to some cause after the operation, and furthermore since no irreversible deformities occur, the lens may be stably maintained at the proper position in the capsule. The number 19 in the figure indicates an anterior capsular incision.

A method for the production of the intraocular lens 10 of the present example will now be described.

Plate or button-shaped objects made of PMMA obtained by (co)polymerization of MMA as the main component are first cut out using a milling machine (milling lathe) to obtain pieces having the rough planar shape of the intraocular lens according to the present example, and then the back surface and the front surface turned in that order using an NC lathe (numerically controlled lathe), and finally the lens subjected to tumble grinding (barrel grinding) to obtain an intraocular lens 10 according to the present example.

### Example 2

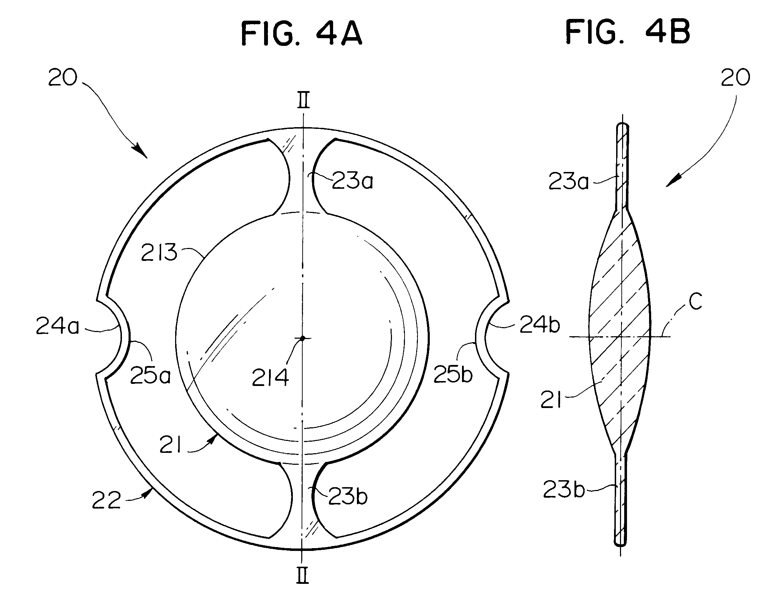

The intraocular lens 20 according to the present example, as shown in Fig. 4 (A) and (B), is the intraocular lens 10 in the above mentioned Example 1, but wherein the angle of inclination α of the connecting sections 13a and 13b is 0° with respect to the plane 115 which passes through the lens center of the optic 11 perpendicular to the optical axis C. That is, the optic 21, connecting sections 23 (23a, 23b), haptic 22 and grips 24 (24a, 24b) of the intraocular lens 20 are all constructed so as to be positioned on the same plane. When the haptic 22 of this intraocular lens 20 is deflected by pinching the grips 24a and 24b with forceps, then the inside surfaces 25a and 25b of the haptic 22 provided with the grips 24a and 24b may come into contact with the outer periphery 213 of the optic 21, to prevent further deformities of the above mentioned haptic 22. However, even in this condition, since the minimum diameter of the intraocular lens after deformation of the haptic may be reduced to about 7.5 mm, or much smaller in comparison with a total diameter of the intraocular lens of 10 mm prior to deformation thereof, intraocular insertion is facilitated.

In the case of the intraocular lens 20 according to the present example as well, when the grips 24a and 24b are pinched with forceps to deflect the haptic 22, deformities such as shown in Fig. 2 may be elastically produced by forcibly applying force in the direction of the optical axis, as in the case of the intraocular lens 10 in Example 1. In this case, the minimum diameter during deformation of the intraocular lens 20 may be reduced to measurements roughly equal to the diameter of the optic, or 6.0 mm, and intraoptical insertion is greatly facilitated.

### Example 3

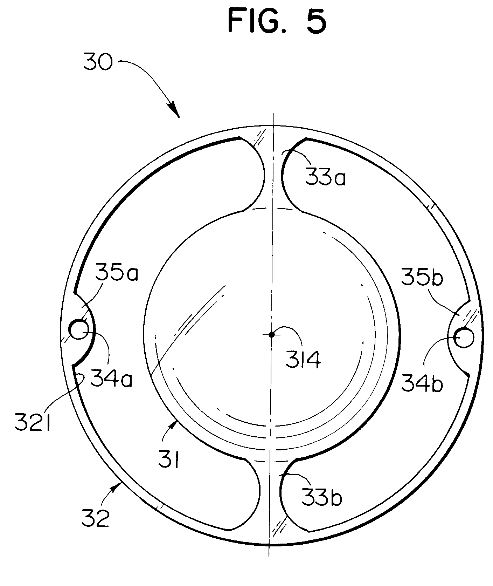

The intraocular lens 30 according to the present example, as shown in Fig. 5, is the intraocular lens 20 in the above mentioned Example 2 (angle of inclination α = 0°), but wherein semi-circular plate-shaped protrusions 35a, 35b having perforations 34a, 34b are provided as grips on the inner surface 321 of the haptic 32. The perforations 34a and 34b are provided for insertion of a handling tool such as, for example, forceps or the like, and the diameter thereof is preferably 0.3 ∼ 3 mm. In the intraocular lens 30 shown in Fig. 5, perforations 34a, 34b of approximately 0.4 mm are constructed. Except for the construction of the above mentioned grips, this intraocular lens 30 is exactly the same as the intraocular lens 20 in Example 2, and if similar measurements are also made, then there will be substantially no difference between the effects of the two.

As a modification of Example 3, an intraocular lens 40 with larger measurements may be made. The measurements of this intraocular lens 40 may be, for example, a total diameter of 11.0 mm, a diameter of the optic of 7.0 mm, a center thickness of the optic of 0.95 mm, a thickness of the haptic of 0.16 mm, a width of the haptic of 0.12 mm, and a diameter of the grip perforations of 0.5 mm. The intraocular lens 40 constructed in this manner is suitable for implantation of an intraocular lens after complete extraction of the lenticular capsule. That is, if the intraocular lens cannot be fixed into the capsule, the intraocular lens is fixed onto the ciliary fossa by suture fixation, but the safety of the above mentioned intraocular lens 40 is considerably improved during intraocular maintenance if the suture fixation is made between the grip perforations and the ciliary fossa using a suture.

### Example 4

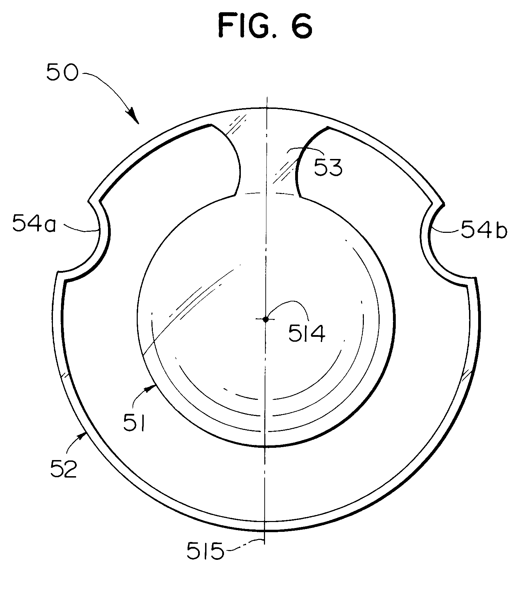

The intraocular lens 50 according to the present example, as shown in Fig. 6, is the intraocular lens 20 in the above mentioned Example 2 (angle of inclination α = 0°), but with only one connecting section 53 and provided with grips 54a and 54b symmetrical with respect to the optical axis surface 515 formed by a straight line connecting the above mentioned connecting section 53 and the lens center 514. Furthermore, the area (or volume) of the connecting section 53 is increased sufficiently to maintain the positional and connection relationship between the optic 51 and the haptic 52 in the same manner as in the intraocular lens 20 with a single connecting section, and the grips 54a and 54b are constructed closer to the connecting section 53. This intraocular lens 50 is substantially the same as the intraocular lens 20 in Example 2, except in the above mentioned points, and there will be substantially no difference between the effects of the two.

### Example 5

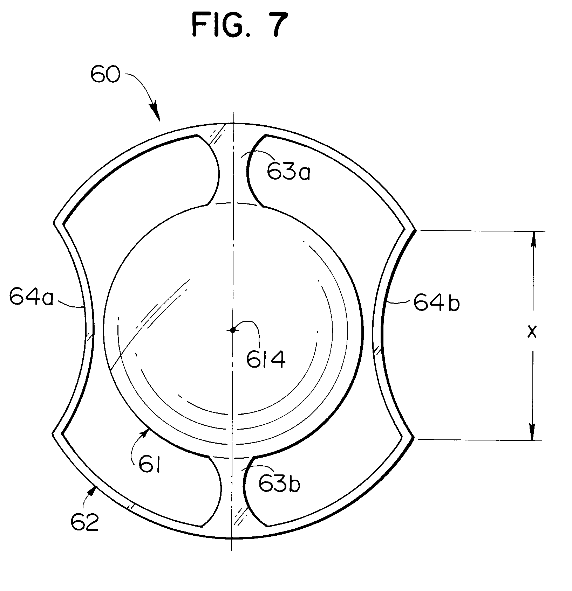

The intraocular lens 60 according to the present example, as shown in Fig. 7, is the intraocular lens 20 in the above mentioned Example 2 (angle of inclination α = 0°), but with the measurements of the grips 64a and 64b increased to occupy approximately 1/3 of the total length of the haptic 62. The curvature radius of the grips of this intraocular lens 60 is 5 mm, and the diameter x of the concave opening is also approximately 5 mm.

In the case of the intraocular lens 60 according to the present example as well, similar to the intraocular lens in Example 2, the haptic 62 may be elastically deformed by pinching by forcible application of a force in the direction of the optical axis. In addition, although the contact area of the haptic 62 with the intracapsular tissue of the lenticular capsule is reduced to approximately 2/3 of the total length of the haptic, it was confirmed that it may be stably maintained in the capsule.

An explanation regarding the intraocular lens according to the present invention has been provided with reference to the Examples, but the shape of the optic of the intraocular lens according to the present invention is not limited to the biconvex intraocular lenses described in the above mentioned Examples, and the intraocular lens to be used may be planoconvex, meniscus-shaped, etc.

In addition, the shape of the grips of the intraocular lens according to the present invention is not particularly limited to the shapes described in the above mentioned Examples, and any one may be used providing it is suitable to the measurements, shape, etc. of the handling tool, for example, forceps or the like, examples of grips which may be used including wedge-shaped and U-shaped grips, etc. Also, regarding the number of grips, the above mentioned Examples all indicate a single pair thereof, but there may be at least one pair (that is, at least two) at a position symmetrical to the optical axis of the optic or at a position symmetrical to the optical axis cross-section of the optic.

The material of the intraocular lens according to the present invention is PMMA in all of the above mentioned Examples, but it is not particularly limited to PMMA, and may be a bioadabtable transparent hard resin which has been publicly known as a material for intraocular lenses, examples thereof including fluorine-containing (meth)acrylate, silicon meth(acrylate), alkyl (meth)acrylate, polycarbonate, etc. According to the present invention, polymethyl methacrylate includes not only homopolymers of methyl methacrylate, but also cross-linked polymethyl methacrylates which are copolymers of methyl methacrylate with an appropriate cross-linking agent, for example, a di- or poly(meth)acrylate of a diol or polyol, such as ethylene glycol dimethycrylate, triethylene glycol dimethycrylate or the like. In addition, an ultraviolet absorber and/or coloring agent, etc. may be added to the polymethyl methacrylates if desired.

An intraocular lens according to the present invention may be easily grasped at the haptic during operations using grips provided on the haptic, and by deflecting of the haptic intraocular insertion is facilitated. Thus the physical burden on the surgeon is alleviated. Furthermore, since insertion may be made through a slit smaller than the entire diameter or with roughly the same diameter as the optic, less inflammation occurs after the operation, and corneal astigmatism may be reduced.

Furthermore, the haptic of an intraocular lens according to the present invention has an annular shape, and therefore the lens itself may be fixed at the center, and high visual functions may be restored without moving the optical center, while the lens may be stably maintained over a long period of time at a prescribed intraocular position.

## Claims

1. An intraocular lens having an optic, a haptic and at least one connecting section connecting the optic and the haptic, said haptic encircling the periphery of the side rim of the optic in a continuous annular shape of substantially 360° along the side rim of said optic, wherein at least one pair of grips for gripping said haptic in an inwardly deflecting manner are provided at points on said haptic symmetrical to the optical axis of said optic or at points on said haptic symmetrical to the optical axis surface of said optic.

2. The intraocular lens according to Claim 1, wherein the grips are concave sections which open towards the outside surface of the haptic.

3. The intraocular lens according to Claim 1, wherein the grips are perforated convex sections which extrude towards the inside surface of the haptic.

4. The intraocular lens according to Claim l, wherein the shape of the plane surface of the connecting sections extends in both directions to increase the area (or volume) of the periphery of the points of contact with the optic and the haptic, respectively.

5. The intraocular lens according to Claim 1, wherein a pair of grips are provided on the haptic along the diameter perpendicular to the diameter connecting the connecting sections.

6. The intraocular lens according to Claim 2, wherein only one connecting section is provided and a pair of grips are constructed closer to the connecting section on the haptic.

7. The introcular lens according to Claim 2, wherein the shape of the grip is semi-circular, wedge-shaped or U-shaped.
